# EUROPEAN PATENT SPECIFICATION

(11) **EP 2 632 300 B1**
(45) Date of publication and mention of the grant of the patent: **24.01.2018**
(21) Application number: 11758560.4
(22) Date of filing: 10.08.2011
(51) Int. Cl.: A47G 9/10, A61H 1/02

(54) **CERVICAL PILLOW FOR TREATMENT OF CERVICAL SPINE DISEASES**
HALSWIRBELPOLSTER ZUR BEHANDLUNG VON ERKRANKUNGEN DER HALSWIRBELSÄULE
OREILLER CERVICAL POUR LE TRAITEMENT DE MALADIES DE LA COLONNE CERVICALE

(30) Priority: 20.09.2010 VN 201000198 U
(43) Date of publication of application: 04.09.2013
(73) Proprietor: Pham Thi Kim, Loan, Ho Chi Minh City (VN)
(72) Inventor: Pham Thi Kim, Loan, Ho Chi Minh City (VN)
(74) Representative: advotec.
(86) International application number: PCT/IB2011/053563
(87) International publication number: WO 2012/038846

(56) References cited:
- DE-A1- 10 140 105
- DE-U1- 9 210 058
- JP-A- 2004 129 885
- JP-A- 2011 067 260
- KR-A- 20110 015 230

## Description

### FIELD OF THE INVENTION

The present invention generally relates to a device for treatment of cervical spine diseases and more particularly, to a cervical pillow for treatment of cervical spine diseases.

### BACKGROUND OF THE INVENTION

Most muscles of the body relax completely when they are not being used; however, muscles in the neck have to work non-stop in order to keep the head in its place. The human neck is constantly fighting against gravity. It also has to bend, lift, turn, and twist resulting in the discs and the facet joints to degenerate. As the discs become thinner, the spaces between the vertebrae become narrower. Sustained contraction, hypertonicity, and motion restriction of the atypical and typical cervical vertebrae, C0-C2 and C2-T1, respectively, can lead to headache and neck pain, and more severely, cervical spine diseases including cervical kyphosis, straight cervical spine, and cervical herniated disc. Several attempts have been made to treat these cervical spine diseases, some of which were artificial cervical disc replacement, therapeutic drugs, and chiropractic care. However, artificial cervical disc replacement is not for everyone, especially not for those with osteoporosis, joint disease, and allergy to stainless steel. In addition, artificial cervical disc replacement requires a discectomy, which may lead to other complications such as infection, excessive bleeding, and damages to nerves, spinal cord, esophagus or vocal cord. Drugs such as acetaminophen and NSAIDs can reduce neck pain; unfortunately, they are only temporary. In addition to the drugs limited ability to treat cervical spine diseases, they cause liver and kidney damage, gastrointestinal bleeding, and ulcers. Chiropractic care seems to be a safer option of the three treatments. Chiropractors use spinal effective manipulation techniques, such as specific spinal manipulation or instrument-assisted manipulation, to treat patients. However, in the process of treating patients, there is a risk of putting excessive forces on the patients' spine that may lead to damaging it. On the other hand, if the chiropractors treat the diseases with totally safe thus much less intensive forces, the treatment period is normally very lengthy. The treatment period is even prolonged given the fact that patients can only spend one to two hours a day with the chiropractors. To treat the diseases safely and to shorten the treatment period, it becomes necessary to have a device that can replicate the safe forces applied by the chiropractors, and that is handy enough for patients to use it whenever needed in order to increase the treatment time each day, thus to shorten the treatment period.

The Olson patent (U.S. Pat. No. 5,644,809) describes a pillow for maintaining proper cervical and thoracic spinal alignment of people as they sleep. This pillow, however, does not have the functions for treating cervical spine diseases.

The Chang-Ho patent application (Publication No. WO/2009/148215) describes a pillow for protecting the cervical vertebrae by maintaining proper head and cervical vertebrae height. This pillow, however, does not have the functions for treating cervical spine diseases.

Finally, the Sang-Hyun patent application (Publication No. WO/2007/091863) describes a pillow for supporting a user's head, neck, shoulders and back. The pillow relaxes the joints of bones and muscles when the user lies on the pillow to strengthen the vertebrae, the nervous tissue and blood vessels, thereby preventing various diseases. This pillow, however, also does not have functions of treating cervical spine diseases.

Patent application DE9210058U1 discloses a cervical pillow according to the preamble of claim 1.

### SUMMARY OF THE INVENTION

The objective of the present invention is to provide a cervical pillow that treats cervical spine diseases. These diseases include cervical kyphosis, straight cervical spine, and cervical herniated disc.

According to the present invention, a cervical pillow for treatment of cervical spine diseases includes a padded member made of resilient materials such as foam, rubber, or cotton. The padded member has a rectangular flat bottom side, vertically flat left and right sides, a rounded front side, a thin back side, and a wavy top side that has a flat portion connected to the front side, a slightly concave portion in the middle, and a very convex portion connecting to the back side. The cervical pillow further includes a solid block made of high-strength materials such as plastic or compressed rubber. This solid block is embedded within the padded member along its back side and under the very convex portion of its top side. The solid block has a nearly rectangular flat base surface and inverted T-shape cross section, wherein said very convex portion of the top side and said solid block together create an elevated firm area with respect to said flat portion configured to push the spinal discs of a user's cervical spine back into their original places

The very convex portion of the top side together with the solid block create an elevated firm area. When a patient lay down in a supine position in which the occipital region of his head is on the slightly concave portion of the top side and his neck is on the elevated firm area, the elevated firm area is, thanks to its very convex shape, capable of generating a strong but safe reaction force against the gravity forces from the head and body. Obviously, the reaction force is placed on his cervical spine. Given that, the elevated firm area is able to treat cervical spine diseases by gradually adjusting the straight or kyphotic cervical spine back to its natural inward curvature. With the same mechanism, the elevated firm area is also able to treat cervical herniated discs by gradually pushing the discs back into their original places.

### BRIEF DESCRIPTION OF THE DRAWINGS

The invention will be better understood when consideration is given to the following detailed description thereof Such description makes reference to the annexed drawings wherein:
FIG. 1 is a perspective left-back view of a cervical pillow for treatment of cervical spine diseases according to the invention.
FIG. 2 is a perspective right-back view of the pillow shown in FIG. 1.
FIG. 3 is a perspective right-front view of the pillow shown in FIG. 1.
FIG. 4 is a perspective left-back view of the pillow shown in FIG. 1.
FIG. 5 is a cross-section side view of the pillow shown in FIG. 4 taken along lines A-A.
FIG. 6 is a cross-section front view of the pillow shown in FIG. 4 taken along lines B-B.
FIG. 7 shows a person in a supine position lying on the pillow shown in FIG. 1

### DETAILED DESCRIPTION OF THE INVENTION

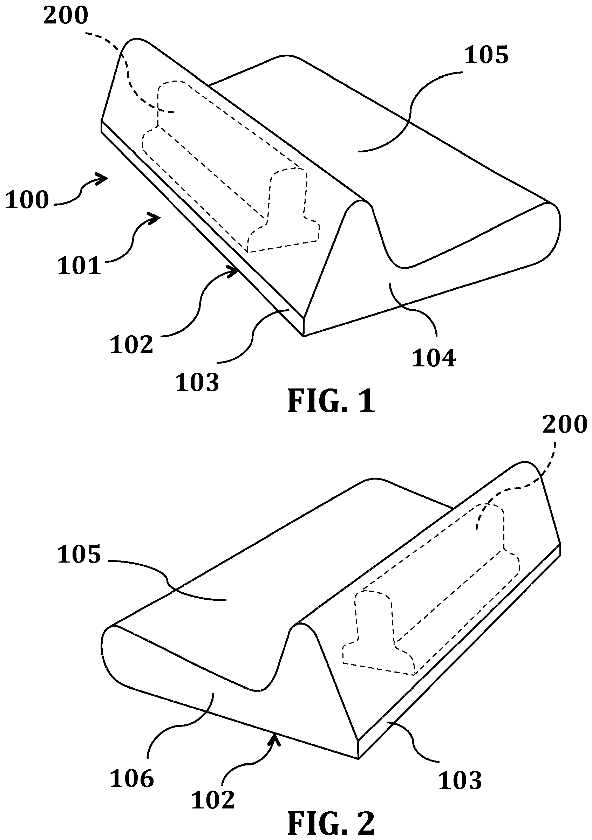

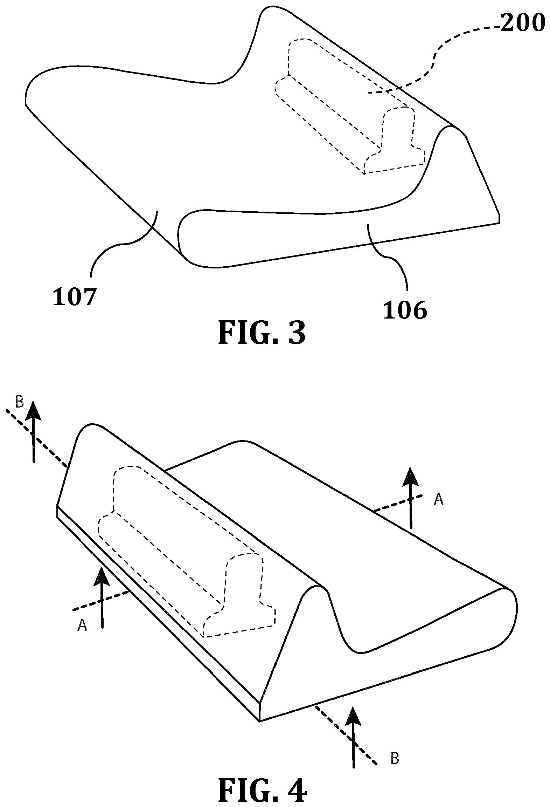

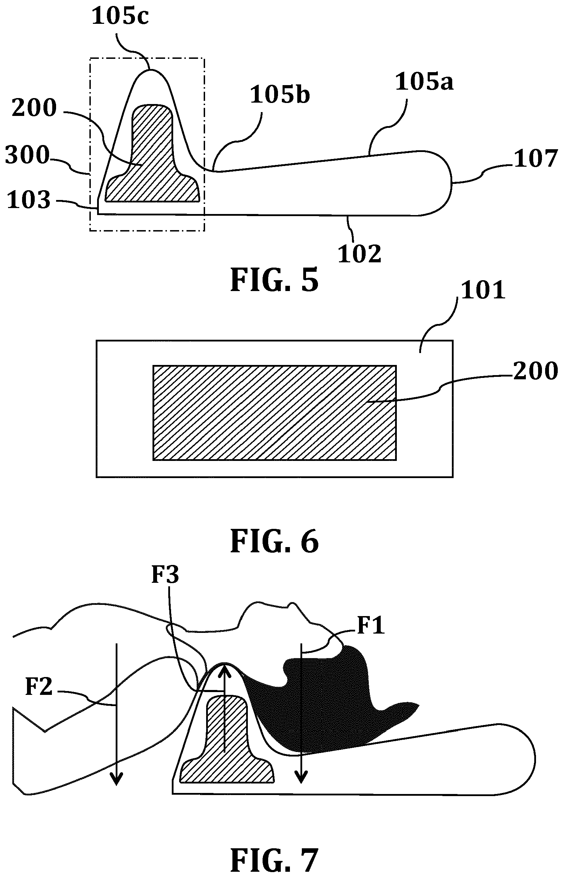

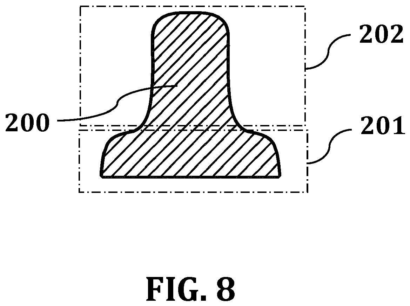

FIGS. 1, 2, 3 and 4 show perspective views of a cervical pillow for treatment of cervical spine diseases 100, which comprises a padded member 101. FIGS. 5 and 6 show cross-section views of a cervical pillow for treatment of cervical spine diseases 100. The padded member 101 comprises rectangular flat bottom side 102, a vertically flat left side 104, a vertically flat right side 106, a rounded front side 107, a thin back side 103, and a wavy top side 105 that has a flat portion 105a connecting to the front side 107 and a slightly concave portion 105b in the middle and a very convex portion 105c connecting to the back side 103, and further includes a nearly rectangular solid block 200 made of high-strength materials such as solid foam, plastic, or compress rubber, inserted inside the padded member 101 along its back side 103 and under the very convex portion 105c of its top side 105. The solid block 200 has a nearly rectangular flat base surface and inverted T-shape cross section, wherein said convex portion 105c of the top side 105 and said solid block 200 together create an elevated firm area 300 with respect to said flat portion 105a configured to push the spinal discs of a user's cervical spine back into their original places.

The very convex portion 105c of the top side 105 together with the solid block 200 create an elevated firm area 300. When a patient lay down in a supine position in which the occipital region of his head is on the slightly concave portion 105b of the top side 105 and his neck is on the elevated firm area 300 as shown in FIG. 7, the elevated firm area 300 is, thanks to its very convex shape, capable of generating a strong but safe reaction force F3 against the gravity force F1 from the head and the gravity force F2 from the body. Obviously, the reaction force F3 is placed on his cervical spine. Given that, the elevated firm area is able to treat cervical spine diseases by gradually adjusting a straight or kyphotic cervical spine back to its natural inward curvature. With the same mechanism, the elevated firm area 300 is also able to treat cervical herniated discs by gradually pushing the discs back into their original places. In this case, a user needs to place his neck onto the elevated firm area in the manner that the herniated disc is right on the peak of the elevated firm area 300.

## Claims

1. A cervical pillow (100) including a padded member (101) made of resilient materials such as foam, rubber, or cotton, wherein the padded member (101) has a rectangular flat bottom side (102), vertically flat left side (104) and a vertically flat right side (106), a rounded front side (107), a thin back side (103), and a wavy top side (105) that has a flat portion (105a) connected to the front side (107), a slightly concave portion (105b) in the middle, and a very convex portion (105c) connected to the back side (103), said cervical pillow (100) further includes a solid block (200) made of high-strength materials such as plastic or compressed rubber, wherein said solid block (200) is embedded within the padded member (101) along its back side (103) and under the very convex portion (105c) of its top side (105), **characterized in that** the solid block (200) has a nearly rectangular flat base surface and inverted T-shape cross-section, wherein said very convex portion (105c) of the top side (105) and said solid block (200) together create an elevated firm area (300) with respect to said flat portion (105a) configured to push the spinal discs of a user's cervical spine back into their original places.

## Patentansprüche

1. Nackenstützkissen (100), umfassend ein gepolstertes Element (101) aus federnden Materialien, wie Schaum, Gummi oder Baumwolle, wobei das gepolsterte Element (101) eine rechteckige flache Unterseite (102), eine senkrecht flache linke Seite (104) und eine senkrecht flache rechte Seite (106), eine abgerundete Vorderseite (107), eine schmale Rückseite (103) und eine gewellte Oberseite (105) aufweist, welche einen mit der Vorderseite (107) verbundenen flachen Abschnitt (105a), einen geringfügig konkaven Abschnitt (105b) in der Mitte und einen mit der Rückseite (103) verbundenen stark konvexen Abschnitt (105c) aufweist, wobei das Nackenstützkissen (100) weiterhin einen soliden Block (200) aus hochfesten Materialien, wie Plastik und komprimiertem Gummi, aufweist, wobei der solide Block (200) entlang der Rückseite (103) in dem gepolstertem Element (101) und unterhalb des stark konvexen Abschnitts (105c) der Oberseite (105) eingebettet ist, **dadurch gekennzeichnet,**
**dass** der solide Block (200) eine nahezu rechteckige flache Grundfläche und einen umgedreht T-förmigen Querschnitt aufweist, wobei der stark konvexe Abschnitt (105c) der Oberseite (105) und der solide Block (200) gemeinsam einen erhöhten festen Bereich (300) gegenüber dem flachen Abschnitts (105a) ausbilden, wobei der erhöhte feste Bereich (300) zum Verschieben der Bandscheiben der Halswirbelsäule eines Nutzers in ihre Ausgangsposition ausgebildet ist.

## Revendications

1. Oreiller (100) cervical comprenant un élément (101) rembourré fait des matériaux résilients, par exemple la mousse, le caoutchouc out le coton, ledit élément (101) rembourré ayant un côté (102) inférieur plat rectangulaire, un côté (104) à gauche verticalement plat et un côté (106) à droite verticalement plat, un côté (107) avant arrondi, un côté (103) arrière mince et un côté (105) supérieure ondulé qui a une partie (105a) plate reliée au côté (107) avant, une partie (105b) légèrement concave disposée dans le centre et une partie (105c) très convexe reliée au côté (103) arrière, ledit oreiller (100) cervical comprenant en outre un bloc (200) solide fait des matériaux hautement résistants, par exemple le plastique ou le caoutchouc comprimé, ledit bloc (200) solide étant encastré dans l'élément (101) rembourré le long de son côté (103) arrière et dessous la partie (105c) très convexe de son côté (105) supérieur,
**caractérisé en ce que**
le bloc (200) solide a une surface de base presque rectangulaire et une section transversale en forme de T inversé, ladite partie (105c) très convexe du côté (105) supérieur et le bloc (200) solide forment ensemble une zone (300) ferme élevée par rapport à ladite partie (105a) plate configurée à presser les disques intervertébrales du rachis cervical d'un utilisateur dans leurs positions initiales.
